# EUROPEAN PATENT SPECIFICATION

(11) **EP 1 969 377 B1**
(45) Date of publication and mention of the grant of the patent: **22.05.2013**
(21) Application number: 07700319.2
(22) Date of filing: 03.01.2007
(51) Int. Cl.: G01N 33/94

(54) **ASSAY, KIT AND APPARATUS FOR DETECTION OF ARTEMISININ DERIVATIVES**
TESTVERFAHREN, KIT UND VORRICHTUNG ZUM NACHWEIS VON ARTEMISININ-DERIVATEN
DOSAGE, KIT ET APPAREIL POUR DÉTECTER DES DÉRIVÉS D'ARTÉMISININE

(30) Priority: 03.01.2006 GB 0600031
(43) Date of publication of application: 17.09.2008
(73) Proprietor: London School of Hygiene and Tropical Medicine, London WC1E 7HT (GB)
(72) Inventor: KAUR, Harparkash, London WC1E 7HT (GB); IOSET, Jean-Robert, CH-1006 Lausanne (CH)
(74) Representative: Fyles, Julie Marie
(86) International application number: PCT/GB2007/000012
(87) International publication number: WO 2007/077444

(56) References cited:
- WO-A-01/79527
- WO-A-03/048766
- DE-U1-202004 009 550
- RU-C1- 2 053 771
- US-A- 4 892 833
- US-A- 5 766 841
- KÄFERSTEIN H ET AL.: "Klinisch-toxikologische Analytik - Suchtstoffe, INTERNET ARTICLE" [Online] 28 May 2005 (2005-05-28), KÜLPMANN R , XP002435697 ISBN: 978-3 527 60301 5 Retrieved from the Internet: URL:www3.interscience.wiley.com/cgi-bin/su mmary/109870231/SUMMARY> page 372 - page 376
- TEWARI S.N. AND SHARMA J. D.: "Spot tests for cannabis materials" INTERNET ARTICLE, [Online] 1 January 1982 (1982-01-01), XP002435694 Retrieved from the Internet: URL:http://www.unodc.org/unodc/bulletin/bu lletin_1982-01-01_3_page010.html> [retrieved on 1982-01-01]
- "Naphthol AS-D Chlorazetat-Esterase und alpha-Naphthylazetat-Esterase" INTERNET-ARTICLE , PRODUCT INFORMATION, [Online] XP002435695 Retrieved from the Internet: URL:http://web.archive.org/web/20051228105 826/http://www.sigmaaldrich.com/sigma/gene ral+information/insert_de_90.pdf> [retrieved on 2003-09]
- GREEN M D ET AL: "Authentication of artemether, artesunate and dihydroartemisinin antimalarial tablets using a simple colorimetric method" TROPICAL MEDICINE AND INTERNATIONAL HEALTH, BLACKWELL SCIENCE, GB, vol. 6, no. 12, December 2001 (2001-12), pages 980-982, XP002249774 ISSN: 1360-2276

## Description

The invention relates to an assay for confirming the presence of an artemisinin derivative in a sample and to apparatus and kits for use in such an assay.

Artemisinin derivatives (ARTS) and artemisinin derivative combinations (ACTs) are recognised to be the most effective antimalarial drugs available at present providing rapid cure with no reports to indicate the emergence of resistant parasitic strains.

All successful drugs are at risk of being counterfeited or produced at lower cost using substandard technology and with lack of quality control. The World Heath Organisation (WHO) has defined counterfeit drugs as those which are "deliberately and fraudulently mislabelled with respect to identity and/or source. Counterfeiting can apply to both branded and generic products and counterfeit products may include products with the correct ingredients or with the wrong ingredients, without active ingredients, with insufficient active ingredients or with fake packaging."[2]. Furthermore, WHO estimates that about 25% of the drugs sold in developing countries are counterfeit and in some countries the rates rise to 40% or higher [3,4]. Substandard drugs are produced with inadequate attention to good manufacturing practices and may have contents and/or dissolution times outside the accepted limits, due to poor quality control [1].

Many developing countries do not have the technical, financial, or human resources required for the application of the set standards for patented products enforced on the major drug companies. It is an unacceptable fact that today, the quality of drugs, and by implication their effectiveness and safety, are less certain for the poorest populations who are attracted by the lower prices of drugs sold outside controlled pharmacies. An added complication is that the poor will buy not just the cheapest product but the smallest pack size available from a variety of outlets. With demand .outstripping production there is further danger of counterfeit drugs taking over the market.

Artesunate is a commonly used artemisinin derivative which is formulated in tablets and for parenteral injection and more recently co-formulated with amodiaquine for administration as combination therapy. An epidemic of fake artesunate tablets in mainland SE Asia has become a very serious public health problem there and has, inevitably, led to many fatalities amongst people who would have otherwise survived their malaria infection. When tested, the artesunate tablets were not found to contain the active compound though they were labeled to resemble the commonly available brand of artesunate manufactured by the Guilin Pharmaceutical Company, Guilin, Peoples Republic of China [5, 6]. Currently, one third to one half of artesunate circulating in mainland SE Asia is counterfeit.

There are serious concerns that this may also become a major problem in Africa [7] as has been demonstrated by a recent study conducted in Kenya and DR Congo [8].

If counterfeit artemisinin derivatives follow the genuine products into Africa, this will precipitate a very serious public health problem. This will result in reduced effectiveness of ARTs and artemisinin derivative combination therapies (ACTs) and loss of confidence in their efficacy. Tax revenues will be lost as counterfeit drugs bypass traditional sales avenues and health systems will face an increase in the cost of monitoring the efficacy and safety of pharmaceuticals [9] as well as the additional economic burden imposed by having to treat infected people.

ARTs are colourless and cannot be detected by simple colorimetric tests because the compounds lack a chromophore (chemical group with UV/visible absorption properties). Several sophisticated techniques. (HPLC/ e.c.d, HPLC/UV post-column derivatisation, LC/MS) have thus been developed but these methods cannot be used in the field and are rather costly for developing countries.

WO 03048766 teaches an assay for detecting both artemisinin and a derivative thereof involving a colourmetric assay but is based on different reagents to the ones described herein.

Thus far one field assay for the detection of counterfeit artesunate has been commercially produced [5]. This method has been modified to include the detection of artemether and dihydroartemisinin [6]. It is a test tube method, relying on the reaction of a commercially available diazonium salt, Fast Red ITR salt (Sigma), with these three ARTS leading to the formation of a yellow coloured product. Unfortunately, the test is not specific as a yellow colour is produced with other drugs e.g. doxycycline and folic acid.

Furthermore, other antimalarials such as amodiaquine, pyronaridine, doxycycline, primaquine, lumefantrine and mepacrine which are yellow in colour will lead to confusion when they are used in combination formulations, such as artesunate/amodiaquine, that are soon to become widely available. It will not be possible to confirm the presence of ARTs in combination therapies, such as amodiaquine/ARTs combination therapy, using the presently available techniques, because the presence of either an ART or amodiaquine will produce a false "positive" yellow result.

Bearing in mind the limitations of the current field test the inventors have developed and validated a user/field friendly method specifically for the authentication of all artemisinin derivatives available to laboratory researchers. The method developed by the inventors is particularly useful because it allows the presence of an ART to be identified and then the positive result confirmed.

In accordance with the invention, there is provided a method of identifying the presence of an artemisinin derivative selected from the group comprising: dihydroartemisinin, artemether, arteether, artesunate, artemisone and artelinic acid in a sample, comprising dissolving the sample in or mixing the same with a solvent, combining the resultant solution with a reagent , dinitrophenylhydrazine, on a support in acidic pH conditions, and observing the sample for a colour change to pink or pink-violet, the colour change being indicative of the presence of the artemisinin derivative.

In another aspect of the invention, there is provided a method of identifying the presence of an artemisinin derivative selected from the group comprising dihydroartemisinin, artemether, arteether, artesunate, artemisone and artelinic acid in a sample, comprising dissolving the sample in or mixing the same with a solvent, combining the resultant solution with the reagent, Fast Blue RR, in acidic pH conditions and observing the sample for a colour change to blue or blue-brown, the colour change being indicative of the presence of an artemisinin derivative and where a colour change is observed, further comprising confirming the presence of an artemisinin derivative by testing the sample with dinitrophenylhydrazine on a support in acidic pH conditions, wherein a colour change to pink or pink-violet is indicative of the presence of an artemisinin derivative; or further comprising testing the sample for the presence of erythromycin, wherein a result demonstrating the absence of erythromycin is indicative of the presence of the artemisinin derivative.

The method of the invention is particularly useful as it allows the presence of an artemisinin derivative to be confirmed very quickly and inexpensively. The presence of an artemisinin derivative is indicated by a colour change. No other anti-malarial drugs have been found to produce the same colour change. Additionally, the method provides two separate reagents that can be used to confirm the presence of an artemisinin derivative. This provides significant advantages, as the reagent may be selected according to which the user prefers or which is most freely available to the user. Alternatively a sample may be tested in two tests, one test with each reagent. This allows confirmation of the result obtained reducing the likelihood of an incorrect result, whether due to a problem with the reagent or user error.

Artemisinin is a well known anti-malarial drug, based on extracts from the herb *Artemisia annua.* The term is known to those skilled in the art. An artemisinin derivative is a compound based on artemisinin that has the same or similar antimalarial properties. In particular, artemisinin derivatives include dihydroartemisinin, artemether, arteether, artesunate, artemisone and artelinic acid. As used herein, the term artemisinin derivative also includes synthetic compounds based on artemisinin, such as OZ277, although OZ277 is not detected by the method of the invention.

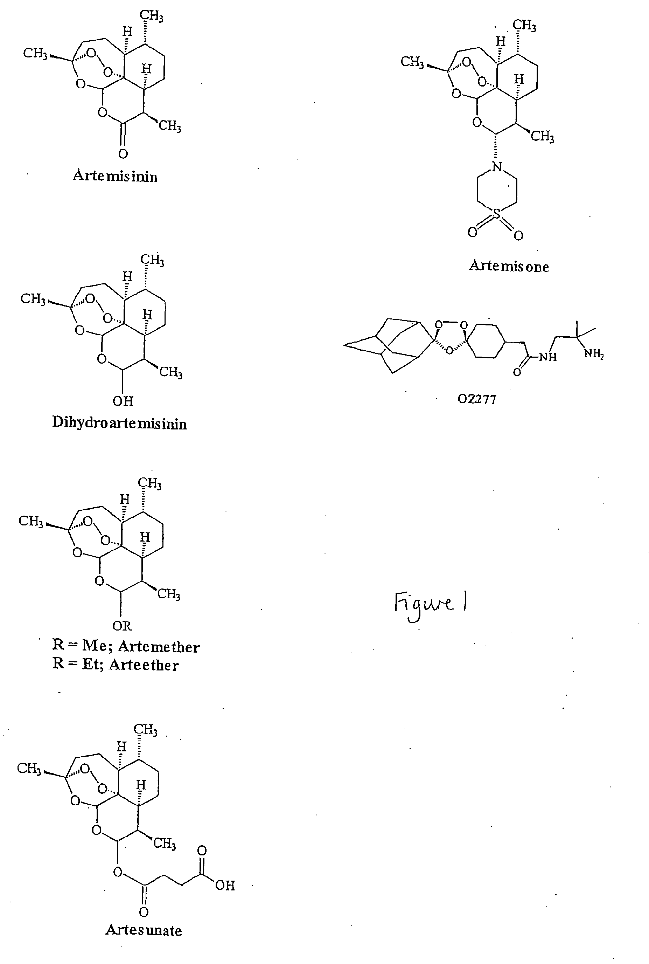

Structures of some artemisinin derivatives are provided in figure 1.

Fast Blue RR is a reagent commonly used as a coupling agent to detect enzyme activities in different media. Briefly, enzyme activities are detected by the conversion of naphthyl acetate into naphthol and the formation of the corresponding purple-coloured diazonium dye with Fast Blue B salt.

Fast Blue RR is the common term for 4'-Amino-2',5-dimethoxybenzanilide and is well known in the art.

Dinitrophenylhydrazine is also known as Brady's reagent and is well known in the art.

Preferably the method is carried out in a strong acidic medium. This allows degradation of the artemisinin derivatives so that their decomposition products can react with the reagents. In particular, the method is preferably carried out at a pH of less than 2, more preferably at a pH of less than 1.

Any appropriate acid may be used to prepare the acidic medium, such as, but not limited to hydrochloric acid, nitric acid, phosphoric acid and sulfuric acid. Preferably hydrochloric acid is used.

Any other appropriate medium could also be used.

The term "sample" includes any sample that a user may wish to test for the presence of an artemisinin derivative. In particular the term sample refers to a tablet or other sample of medicine that purports to contain an artemisinin derivative. The sample may be in any form, for example it may be a liquid or a solid. When in solid form, a user is likely to dissolve the sample in a solvent, especially water or an organic solvent, such as methanol. Alternatively the solid sample may be dissolved directly into the reagent. The sample may also be dissolved in the acid medium before addition of the reagent.

It is preferred that the sample is in solid form, preferably in the form of a crushed tablet. When testing potentially counterfeit tablets, it is useful to crush the tablet and test some of the resulting powder, in order to confirm whether the artemisinin derivative is present throughout the tablet. It is well known for some counterfeiters to coat tablets that do not contain artemisinin derivatives in a thin layer of artemisinin derivative. Tests on the surface of such tablets suggest that they are authentic tablets when, in fact, the amount of active ingredient contained in the tablet is significantly reduced from what it should be.

It is particularly advantageous to dissolve the sample in an organic solvent, especially methanol, ethanol or acetonitrile, particularly when testing a sample of a drug which is likely to contain other components such as excipients because ARTs dissolve in such solvents, but many excipients and drugs do not. This provides an extra selectivity step to remove some compounds. The preferred solvent is methanol.

In order to carry out the method in an acidic medium, and use an organic solvent, the solvent may be acidified. It is particularly useful to use an acidified solvent, because this allows the two preferred method steps to be combined, reducing the overall number of steps and hence time taken to carry out the method. In particular, acidified methanol, such as methanol acidified with hydrochloric acid may be used. Methanol and hydrochloric acid may be used in any appropriate ratio, such as 2:1, 1:1 and 1:2. Any appropriate strength of hydrochloric acid may be used, such as 1M, 2M, 5M or 8M.

The term "sample" also includes a sample obtained from a subject, such as a urine or blood sample. In particular, it might be useful to test a urine sample to check whether a artemisinin derivative is being excreted by the subject in question.

The sample and reagent are preferably combined on a support. A support is any appropriate component on which the sample and reagent may be combined and a colour change observed. It is especially preferred that the support is filter paper or a thin layer chromatography plate (TLC plate). Any appropriate TLC plate may be used, for example a TLC plate with alumina backing.

The reagent and sample may be combined in any appropriate way. For example, where the reagent is in liquid form and the sample in solid form, the sample may be dissolved in the reagent. Alternatively, the sample may be in liquid form, or dissolved in a liquid, before being combined with the reagent.

When the reagent and sample are combined on a support, the two may be combined in any appropriate way. For example, the two may be combined as above and then applied to the support, for example by being sprayed, dropped or poured onto the support, or by the support being dipped into the combination. Alternatively, one of the reagent and sample may be applied to the support and then the other applied. For example, the sample may be applied to the support and then the reagent applied onto the areas of sample, or the reagent could be applied to a support that is then dipped into a dissolved sample. As one skilled in the art would know, there are several methods of combining and applying the sample and reagent, and the above examples are not limiting.

When dinitrophenylhydrazine is used as the reagent, the sample preferably changes colour to yellow or orange when in solution (for comparison reasons), or pink when on a support. When Fast Blue RR is used as the reagent, the sample preferably changes colour to blue.

The inventors have surprisingly found that other antimalarials, excipients and other drugs such as pain killers, antibiotics and antiretrovirals did not produce a positive result using the method of the invention. The majority of the compounds tested did not produce a colour change at all. Some produced a slight colour change, but not to the same extent or same colour as artemisinin derivatives. The method of the invention is specific and sensitive to artemisinin derivatives.
The inventors have tested more than 30 excipients used in the manufacture of antimalarial formulations, 80 drugs (pure substances but also in pills) and 30 antimalarials. No interference has been observed with excipients in either test. No interference has been observed with antimalarials in the dinitrophenylhydrazine DNP assay (both supports) or with the Fast Blue RR assay on TLC.

The inventors have noted that there can be interference from the antibiotic erythromycin as it produces a blue color on both supports when tested with Fast Blue RR. Interference from erythromycin can be confirmed by a colour test and this step may be included in the method. Furthermore, any interference experienced with Fast Blue RR could easily be confirmed as interference, rather than a positive result, by repeating the test using dinitrophenylhydrazine.

As indicated above, the method may also be combined with a colour test for erythromycin, to confirm that a positive result is due to an artemisinin derivative, rather than erythromycin. The method may include reacting the sample with a solution of xanthydrol in a mixture of acetic acid and hydrochloric acid and heating the resultant solution. If erythromycin is present a red colour is observed.

Preferably xanthydrol is used at a concentration of around 0.2g/l. Preferably the acetic acid and hydrochloric acid are combined in a ratio of 1:99.

Alternatively, the method may comprise reacting the sample with a solution of hydrochloric acid. A yellow colour is seen after several minutes if erythromycin is present.

The method preferably includes drying the support. This can be done in any appropriate way, including simply leaving the support to dry at room temperature.

Preferably, the method comprises combining part of the sample with dinitrophenylhydrazine and part of the sample with Fast Blue RR, and observing colour changes in both combinations.

This method is particularly useful as it provides a double confirmation of the presence of an artemisinin derivative, helping avoid false positives.

Also provided is a kit for testing for the presence of an artemisinin derivative selected from the group comprising: dihydroartemisinin, artemether, arteether, artesunate, artemisone and artelinic acid in a sample, comprising a support and a supply of reagents dinitrophenylhydrazine and Fast Blue RR, and an acidified solvent.

Preferably, the kit also comprises instructions on how to test for an artemisinin derivative.

The kit comprises an acidified solvent; as described above, in which the sample to be tested may be dissolved.

The kit may also comprise a guide, such as a colour chart, to assist a user in confirming whether a positive result has been reached. The strength of colour produced during the test is an indicator of the amount of artemisinin derivative that is present. The colour chart may include a number of different strengths of colour to allow a user to gain an idea of the amount of artemisinin derivative present.

The kit may further comprise a positive sample to be run in parallel with the test sample as a control or for comparison.

Additionally provided is an apparatus for testing for the presence of an artemisinin derivative selected from the group comprising: dihydroartemisinin, artemether, arteether, artesunate, artemisone and artelinic acid, comprising a support bearing reagents dinitrophenylhydrazine and Fast Blue RR and an acidified solvent.

The reagents are carried on the support in any way, such that when a sample containing an artemisinin derivative is applied to the support, a colour change may be seen.

At least a part of the apparatus may be coloured, preferably with the colour or colours seen when a positive test result is achieved. A user can then easily compare the colour of the reagent following application of the sample to the colour on the apparatus to confirm the positive result.

The apparatus may be arranged so that the sample may be applied in any way. The sample may be sprayed, dropped or poured onto the area of the apparatus bearing the reagent.

The apparatus is preferably a dipstick. The term dipstick is well known in the art and refers to an apparatus for testing a sample. A dipstick is usually a support normally elongated in shape that is dipped into a liquid sample. The dipstick bears a reagent that changes colour if a particular compound is present in the sample.

The apparatus, such as a dipstick, may have a coating, particularly a coating in which the reagents are imbedded. The coating may be any appropriate coating, such as alumina, cellulose or silica.

The invention will now be described in detail by way of example only.

### EXAMPLES

### Samples preparation

2.5 mg of pure chemicals used as reference drugs, alternatively 5 mg of ground powder obtained from a sample to be analyzed (tablets, capsules etc.) are dissolved in a volume of 200 µl of methanol. The solution is mixed (shaking or other method if available such as vortex or ultrasonic bath) for 30 sec and eventually left on the bench for a couple of minutes (sedimentation).

### Preparations of reagents

**DNP:** 25 mg of 2,4-dinitrophenylhydrazine is dissolved in 50 ml of a 80% methanolic 1M hydrochloric acid (HCl) solution.

**FBS:** 125 mg of Fast Blue RR salt is dissolved in 25 ml of water. This solution is mixed with 25 ml of a 80% methanolic 2M sulfuric acid (H₂SO₄) solution.

### Application of test material on plates/filter paper

Thin layer chromatography (TLC): silica gel 60 F₂₅₄ Al sheets (Merck) are used.
Filter paper: any standard filter paper can be used.

A volume of 5-10 µl of solution (see preparation of samples) is applied on TLC or filter paper using a micropipette or a graduated capillary (solution is applied slowly to avoid large spots).

### The spraying process

Both reagents are sprayed until plates/filter get saturated (wet). TLC/filters are left to dry out at room temperature. Alternatively the reagent may be slowly dropped onto the TLC or filter paper.

### Results observed

DNP: ARTS are detected as pink spots (yellow background) after around 30min-lh at room temperature.

FBS: ARTS are detected as blue spots (white background) after around 1-3h at room temperature.

Both reactions appear with greater rapidity on TLC than on filter paper.

Tables 1, 2 and 3 show the results of testing various antimalarials, excipients and other drugs with the method provided above. The columns marked Green before, Green after, Green mod before and Green mod after refer to the colour of the sample when tested using the method developed by Green *et al.* Green before and after refer to before and after addition of reagents in Green's original method for detecting artesunate, which used water soluble reagents. Green mod before and after refer to before and after addition of reagents in Green's modified method for derivatives which uses organic solvents. The columns FBS, DNP and H₂SO₄, (before and after for each) refer to the colour of the solution on a support before and after addition of Fast Blue Salt, dinitrophenylhydrazine and sulfuric acid respectively. The term "nc" used in some columns means no colour. The term "nt" means not tested. As can be seen, colour changes can be seen with all artemisinin derivatives tested, that are not seen with any other test substance. The two solubility columns refer to solubility in water and in organic solvents.

### REFERENCES

[1] Newton, P., S. Proux, et al. (2001). Fake artesunate in southeast Asia. Lancet 357(9272): 1948-1950.
[2] Newton, P. N., A. Dondorp, et al. (2003). Counterfeit artesunate antimalarials in southeast Asia. Lancet 362 (9378): 169.
[3] GPHF-Minilab^{®}, pdf documents available using the following link: Http://www.gphf.org/web_en/publikationen/manuals.htm
[4] Gabriëls, M., Plaizier-Vercammen, J. (2004). Development of a Reverse-Phase Thin-layer chromatrographic method for artemisinin and its derivatives. Journal of Chromatrographic Science, 42, 341-347.
[5] Green, M. D., D. L. Mount, et al. (2000). "A colorimetric field method to assess the authenticity of drugs sold as the antimalarial artesunate." Journal of Pharmaceutical and Biomedical Analysis 24(1): 65-70.
[6] Green, M. D., D. L. Mount, et al. (2001). "Authentication of artemether, artesunate and dihydroartemisinin antimalarial tablets using a simple colorimetric method." Tropical Medicine & International Health: TM & IH 6(12): 980-982.
[7] (Paul N Newton, Rose McGreaddy, Facundo Fernandez, Michael D Green, Souly Phanouvong, Christopher JM Whitty, Ambrose O. Talisuna, Stephane Proux, Eva Maria Christophel, Grace Malenga, Pratap Singhasivanon, Kalifa Bojang, Harparkash Kaur, Kevin Palmer, Nicholas PJ Day , Brian M Greenwood, François Nosten, Nicholas J White (2006), Manslaughter by fake artesunate in Asia -will Africa become 'infected' next?; PLoS Med. Jun 13;3(6):e197)
[8] Aternkeng MA, Cock de K and Plaizier-Vercammen J (2007). " Quality control of active ingredients in artemisinin-derivative antimalarials within Kenya and DR Congo". Tropical Medicine & International Health, Vol 12, No 1, pp 68-74.
[9] Scheer A, http://www.abnh.com/security/pharm_whitepaper_WEBSITE.pdf,2003.

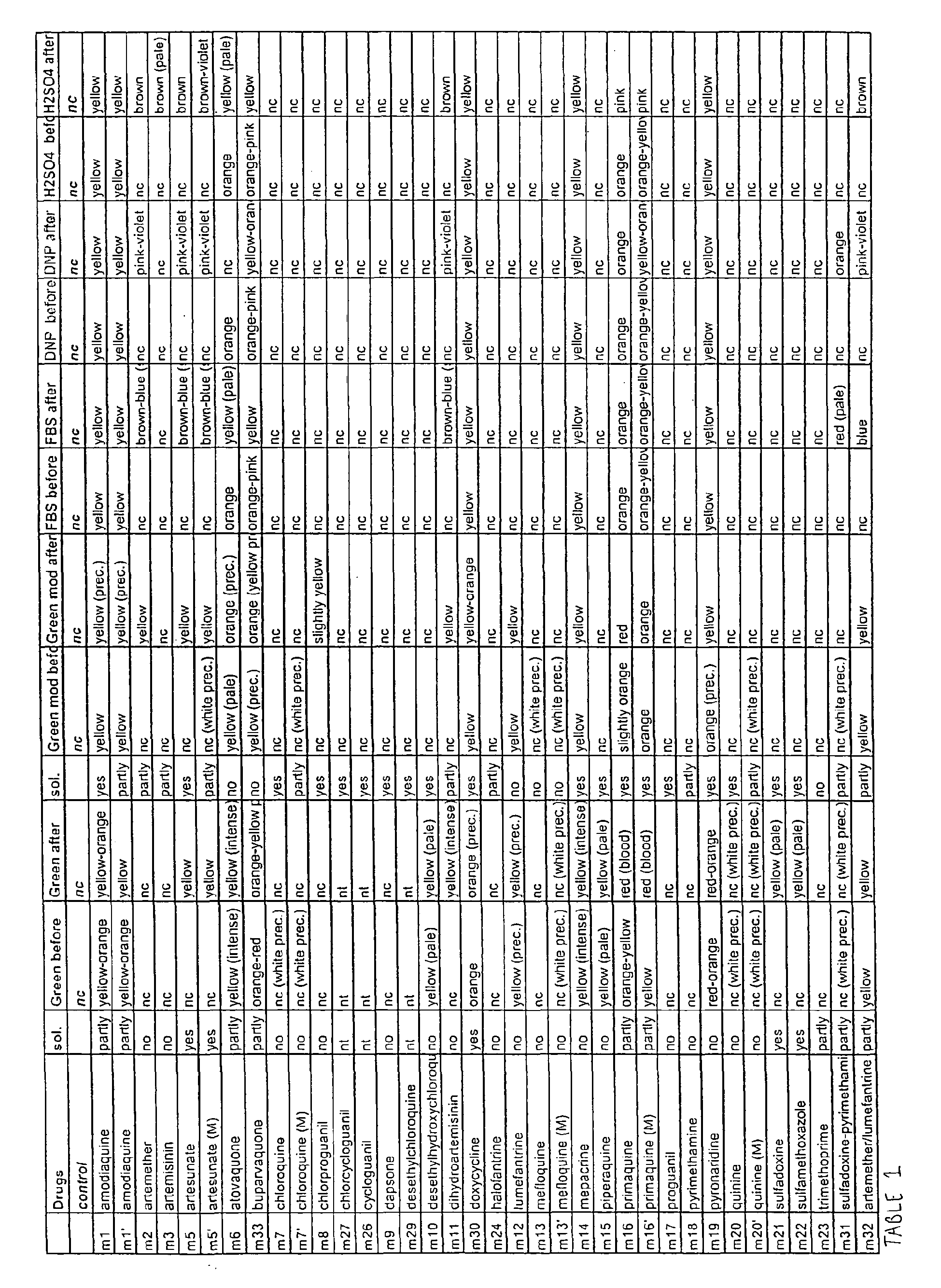

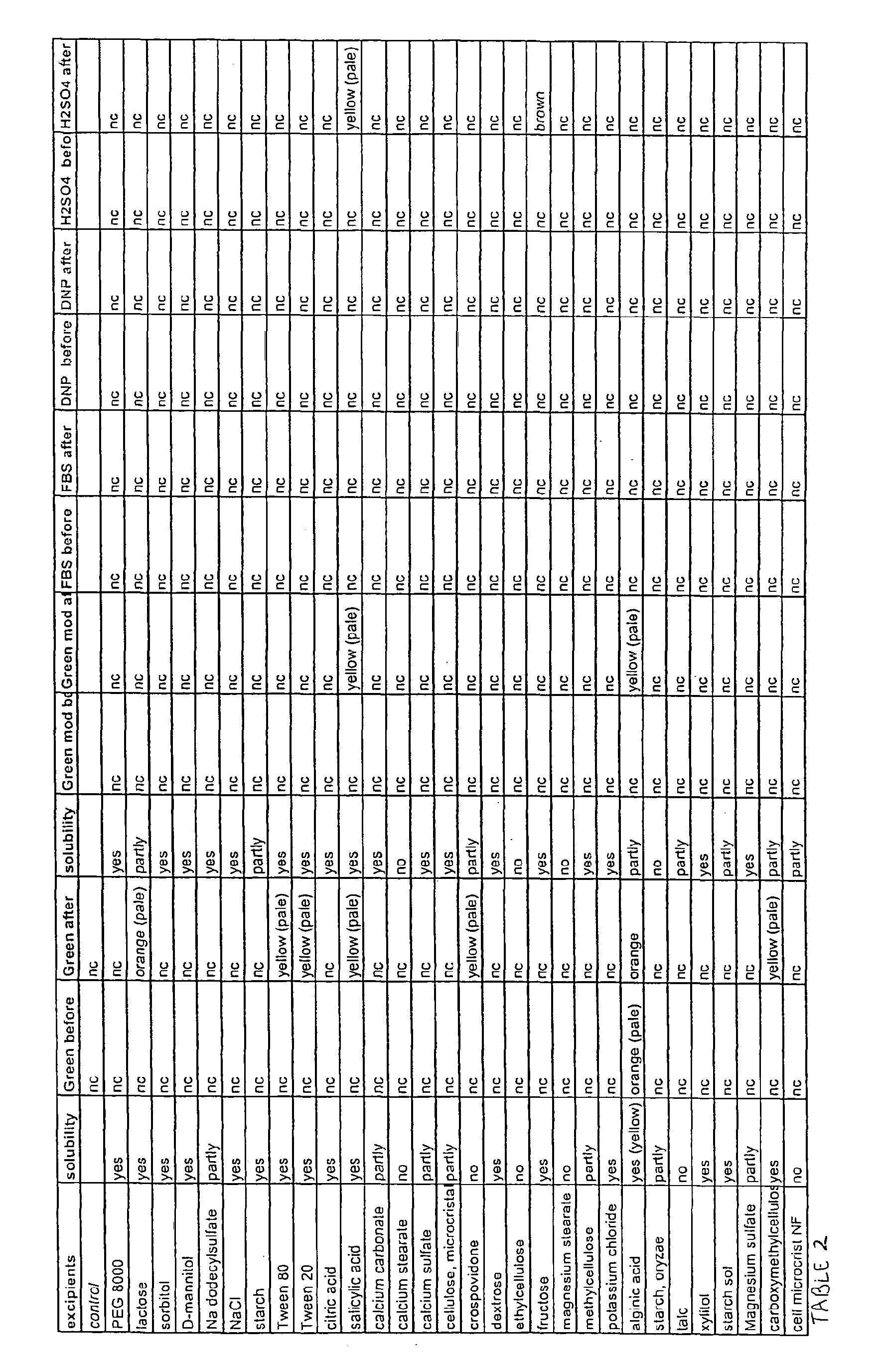

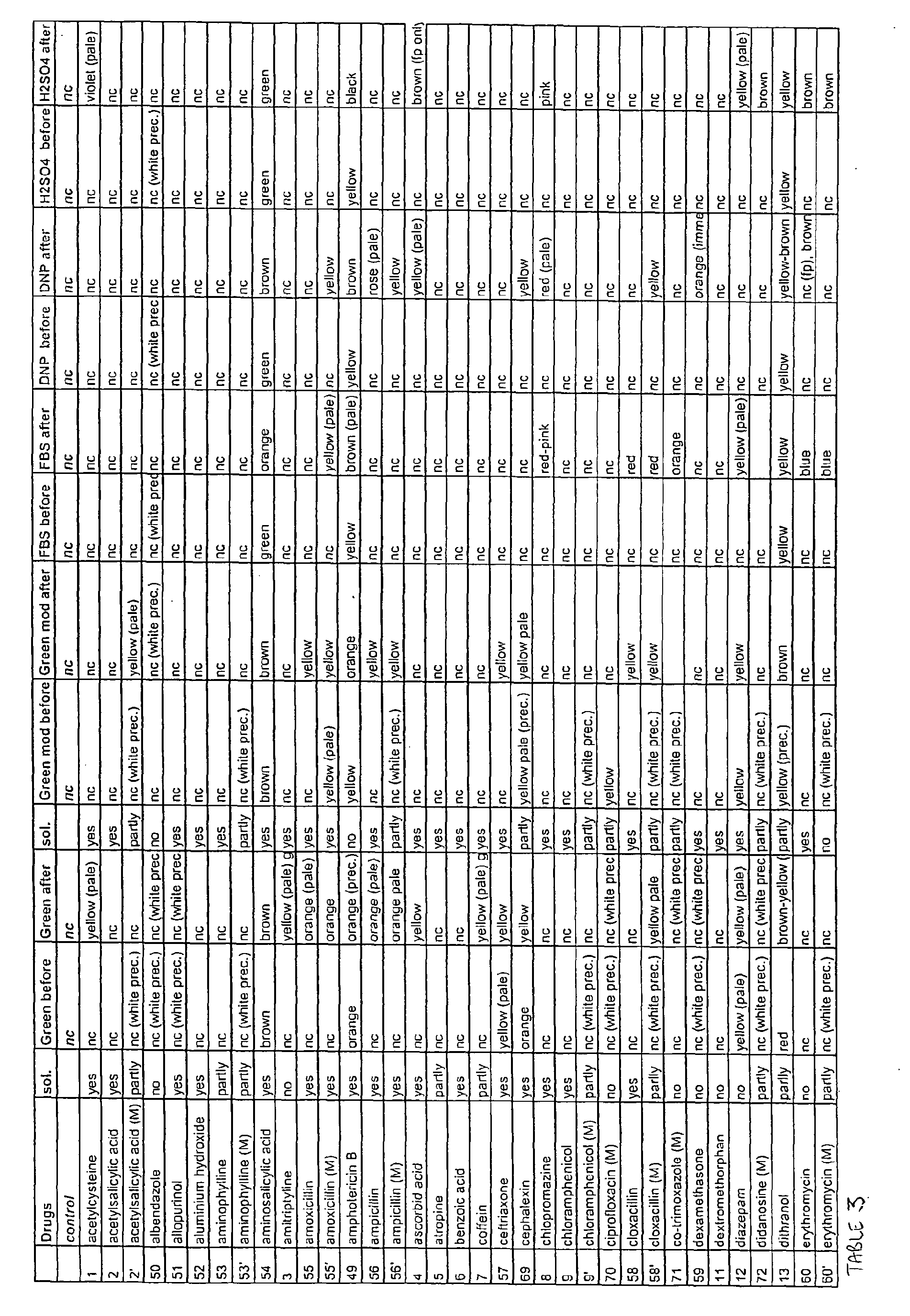

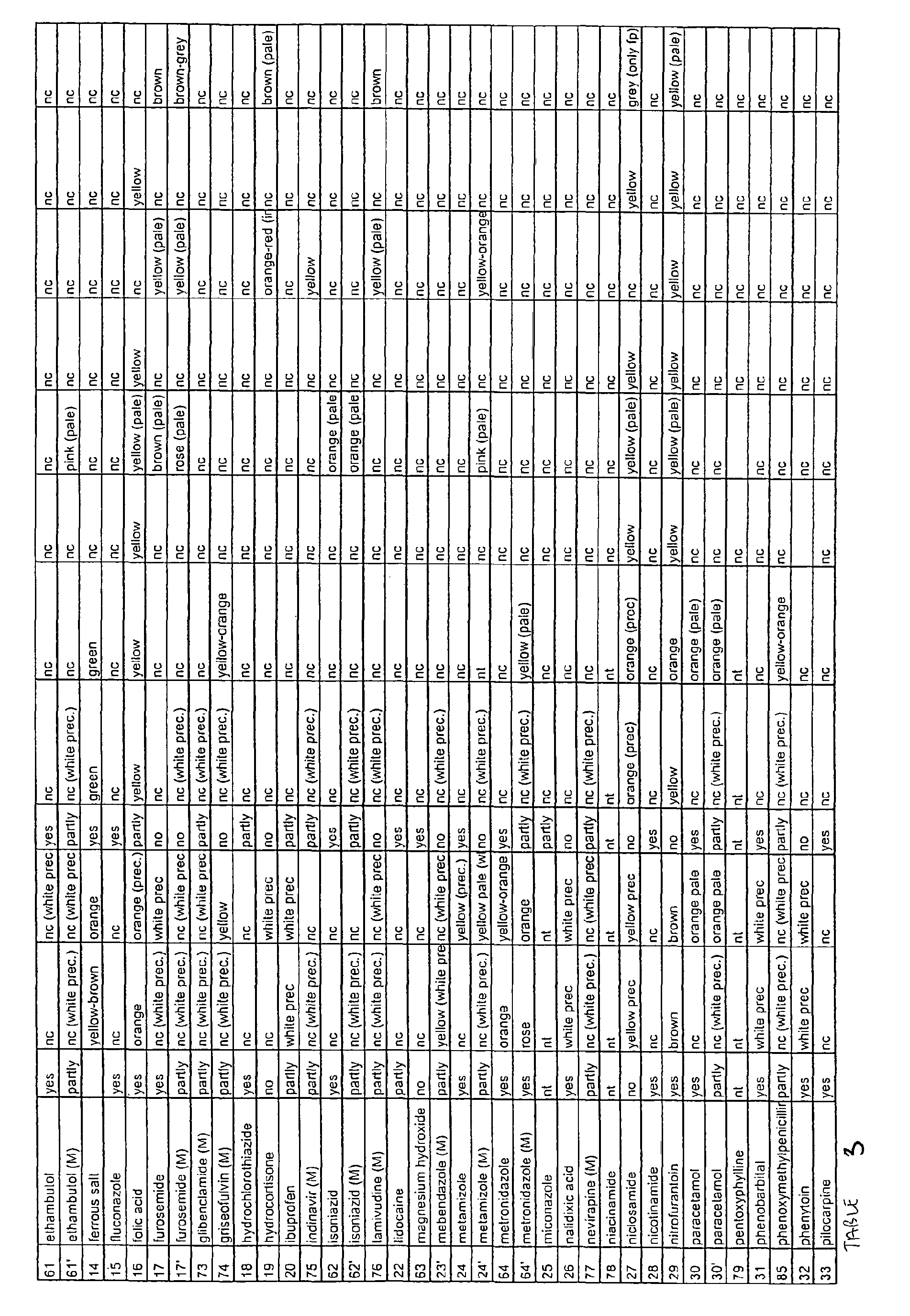

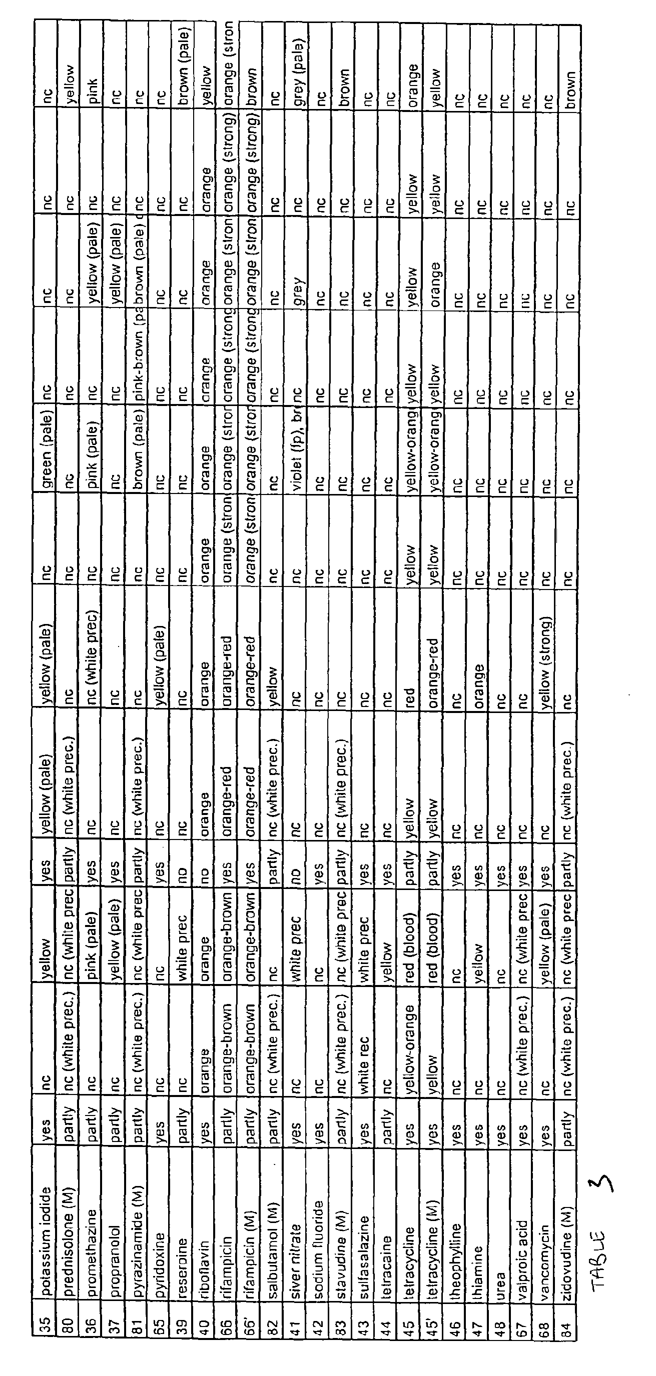

## Claims

1. A method for identifying the presence of an artemisinin derivative selected from the group comprising: dihydroartemisinin, artemether, arteether, artesunate, artemisone and artelinic acid in a sample, comprising dissolving the sample in or mixing the same with a solvent, combining the resultant solution with the reagent, dinitrophenylhydrazine, on a support in acidic pH conditions and observing the solution for a colour change to pink or pink-violet, the colour change being indicative of the presence of the artemisinin derivative.

2. A method of identifying the presence of an artemisinin derivative selected from the group comprising: dihydroartemisinin, artemether, arteether, artesunate, artemisone and artelinic acid in a sample, comprising dissolving the sample in or mixing the same with a solvent, combining the resultant solution with the reagent, Fast Blue RR, in acidic pH conditions and observing the sample for a colour change to blue or blue brown, the colour change being indicative of the presence of the artemisinin derivative and where a colour change is observed, further comprising confirming the presence of an artemisinin derivative by testing the sample with dinitrophenylhydrazine on a support in acidic pH conditions, wherein a colour change to pink or pink-violet is indicative of the presence of the artemisinin derivative; or further comprising testing the sample for the presence of erythromycin, wherein a result demonstrating the absence of erythromycin is indicative of the presence of the artemisinin derivative.

3. The method of claim 2, wherein the test for the presence of erythromycin comprising reacting the sample with a solution of xanthydrol in a mixture of acetic acid and hydrochloric acid, heating the resultant solution and observing the colour of the solution, a red colour being indicative of the presence of erythromycin.

4. The method of claim 2, wherein the test for the presence of erythromycin comprising reacting the sample with a solution of hydrochloric acid and observing the colour of the solution, a yellow colour being indicative of the presence of erythromycin.

5. A method according to any preceding claim, wherein the support is one of filter paper, a thin layer chromatography plate and a dipstick.

6. A kit for testing for the presence of an artemisinin derivative selected from the group comprising: dihydroartemisinin, artemether, arteether, artesunate, artemisone and artelinic acid in a sample comprising a support and a supply of reagents dinitrophenylhydrazine and Fast Blue RR and an acidified solvent.

7. A kit according to claim 6, wherein the support is one of filter paper, a thin layer chromatography plate and a dipstick.

8. An apparatus for testing for the presence of an artemisinin derivative selected from the group comprising: dihydroartemisinin, artemether, arteether, artesunate, artemisone and artelinic acid in a sample comprising a support bearing reagenths-dinitrophenylhydrazine and Fast Blue RR and an acidified solvent.

9. An apparatus according to claim 8, wherein the support is one of filter paper, a thin layer chromatography plate and a dipstick.

## Patentansprüche

1. Verfahren zur Erkennung des Vorhandenseins eines Artemisininderivats ausgewählt aus der Gruppe bestehend aus: Dihydroartemisinin, Artemether, Arteether, Artesunat, Artemison und Artelinsäure in einer Probe, welches das Auflösen der Probe in oder das Mischen selbiger mit einem Lösungsmittel, das Kombinieren der resultierenden Lösung mit dem Reagenz Dinitrophenylhydrazin auf einem Träger unter sauren pH-Bedingungen und das Überprüfen der Lösung auf eine Farbveränderung zu Rosa oder Rosa-Violett umfasst, wobei die Farbveränderung das Vorhandensein des Artemisininderivats anzeigt.

2. Verfahren zur Erkennung des Vorhandenseins eines Artemisininderivats ausgewählt aus der Gruppe bestehend aus: Dihydroartemisinin, Artemether, Arteether, Artesunat, Artemison und Artelinsäure in einer Probe, welches das Auflösen der Probe in oder das Mischen selbiger mit einem Lösungsmittel, das Kombinieren der resultierenden Lösung mit dem Reagenz Fast Blue RR unter sauren pH-Bedingungen und das Überprüfen der Probe auf eine Farbveränderung zu Blau oder Blau-Braun umfasst, wobei die Farbveränderung das Vorhandensein des Artemisininderivats anzeigt und wobei eine Farbveränderung beobachtet wird, das ferner das Bestätigen des Vorhandenseins eines Artemisininderivats durch Testen der Probe mit Dinitrophenylhydrazin auf einem Träger unter sauren pH-Bedingungen umfasst, wobei eine Farbveränderung zu Rosa oder Rosa-Violett das Vorhandensein des Artemisininderivats anzeigt; oder das ferner das Testen der Probe auf das Vorhandensein von Erythromycin umfasst, wobei ein Ergebnis, welches das Fehlen von Erythromycin anzeigt, das Vorhandensein des Artemisininderivats anzeigt.

3. Verfahren nach Anspruch 2, wobei der Test für das Vorhandensein von Erythromycin das Reagieren der Probe mit einer Xanthydrollösung in einer Mischung aus Ethansäure und Chlorwasserstoffsäure, das Erwärmen der resultierenden Lösung und das Überprüfen der Farbe der Lösung umfasst, wobei eine rote Färbung das Vorhandensein von Erythromycin anzeigt.

4. Verfahren nach Anspruch 2, wobei der Test für das Vorhandensein von Erythromycin das Reagieren der Probe mit einer Lösung aus Chlorwasserstoffsäure und das Beobachten der Farbe der Lösung umfasst, wobei eine gelbe Färbung das Vorhandensein von Erythromycin anzeigt.

5. Verfahren nach einem der vorherigen Ansprüche, wobei der Träger eins von Filterpapier, einer Dünnschichtchromatographie-Platte und einem Teststäbchen ist.

6. Kit zum Testen auf das Vorhandensein eines Artemisininderivats ausgewählt aus der Gruppe bestehend aus Dihydroartemisinin, Artemether, Arteether, Artesunat, Artemison und Artelinsäure in einer Probe, das einen Träger und ein Angebot an den Reagenzien Dinitrophenylhydrazin und Fast Blue RR und ein angesäuertes Lösungsmittel umfasst.

7. Kit nach Anspruch 6, wobei der Träger eins von Filterpapier, einer Dünnschichtchromatographieplatte und einem Teststäbchen ist.

8. Vorrichtung zum Testen auf das Vorhandensein eines Artemisininderivats ausgewählt aus der Gruppe bestehend aus Dihydroartemisinin, Artemether, Arteether, Artesunat, Artemison und Artelinsäure in einer Probe, die einen Träger umfasst, der die Reagenzien Dinitrophenylhydrazin und Fast Blue RR und ein angesäuertes Lösungsmittel trägt.

9. Vorrichtung nach Anspruch 8, wobei der Träger eins von Filterpapier, einer Dünnschichtchromatographie-Platte und einem Teststäbchen ist.

## Revendications

1. Procédé pour identifier la présence d'un dérivé d'artémisinine choisi dans le groupe comprenant : la dihydroartémisinine, l'artéméther, l'artééther, l'artésunate, l'artémisone et l'acide artélinique dans un échantillon, comprenant la dissolution de l'échantillon dans un solvant ou le mélange avec celui-ci, la combinaison de la solution obtenue avec le réactif dinitrophénylhydrazine sur un support dans des conditions de pH acide et l'observation d'un changement de couleur de la solution en rose ou en rose-violet, le changement de couleur étant indicateur de la présence du dérivé d'artémisinine.

2. Procédé d'identification de la présence d'un dérivé d'artémisinine choisi dans le groupe comprenant : la dihydroartémisinine, l'artéméther, l'artééther, l'artésunate, l'artémisone et l'acide artélinique dans un échantillon, comprenant la dissolution de l'échantillon dans un solvant ou le mélange avec celui-ci, la combinaison de la solution obtenue avec le réactif Fast Blue RR dans des conditions de pH acide et l'observation d'un changement de couleur de l'échantillon en bleu ou en bleu brun, le changement de couleur étant indicateur de la présence du dérivé d'artémisinine et où un changement de couleur est observé, comprenant en outre la confirmation de la présence d'un dérivé d'artémisinine en testant l'échantillon avec la dinitrophénolhydrazine sur un support dans des conditions de pH acide, dans lequel un changement de couleur en rose ou en rose-violet est indicateur de la présence du dérivé d'artémisinine ; ou comprenant en outre le test de l'échantillon pour la présence de l'érythromycine, dans lequel un résultat démontrant l'absence d'érythromycine étant indicateur de la présence du dérivé d'artémisinine.

3. Procédé selon la revendication 2, dans lequel le test pour la présence d'érythromycine comprend la réaction de l'échantillon avec une solution de xanthydrol dans un mélange d'acide acétique et d'acide chlorhydrique, le chauffage de la solution obtenue et l'observation de la couleur de la solution, une couleur rouge étant indicatrice de la présence d'érythromycine.

4. Procédé selon la revendication 2, dans lequel le test pour la présence d'érythromycine comprend la réaction de l'échantillon avec une solution d'acide chlorhydrique et l'observation de la couleur de la solution, une couleur jaune étant indicatrice de la présence d'érythromycine.

5. Procédé selon l'une quelconque des revendications précédentes, dans lequel le support est un parmi un papier filtre, une plaque de chromatographie sur couche mince et une bandelette réactive.

6. Kit pour tester la présence d'un dérivé d'artémisinine choisi dans le groupe comprenant : la dihydroartémisinine, l'artéméther, l'artééther, l'artésunate, l'artémisone et l'acide artélinique dans un échantillon comprenant un support et un apport des réactifs dinitrophénylhydrazine et Fast Blue RR et un solvant acidifié.

7. Kit selon la revendication 6, dans lequel le support est un parmi un papier filtre, une plaque de chromatographie sur couche mince et une bandelette réactive.

8. Appareil pour tester la présence d'un dérivé d'artémisinine choisi dans le groupe comprenant : la dihydroartémisinine, l'artéméther, l'artééther, l'artésunate, l'artémisone et l'acide artélinique dans un échantillon comprenant un support portant les réactifs dinitrophénylhydrazine et Fast Blue RR et un solvant acidifié.

9. Appareil selon la revendication 8, dans lequel le support est un parmi un papier filtre, une plaque de chromatographie sur couche mince et une bandelette réactive.
